# EUROPEAN PATENT APPLICATION

(11) **EP 4 257 608 A1**
(43) Date of publication of application: **11.10.2023**
(21) Application number: 21903810.6
(22) Date of filing: 07.12.2021
(51) Int. Cl.: C07K 16/28, C07K 14/725, A61P 35/00, A61K 39/00

(54) **ANTIBODY SPECIFIC FOR CD47 AND USE THEREOF**

(30) Priority: 07.12.2020 KR 20200169415
(71) Applicant: Innobation Bio Co., Ltd., Seoul 03929 (KR)
(72) Inventor: KIM, Seung ku, Yongin-si, Gyeonggi-do 16865 (KR); KIM, Ki Tae, Seoul 06148 (KR)
(74) Representative: J A Kemp LLP
(86) International application number: PCT/KR2021/018467
(87) International publication number: WO 2022/124764

(57) **Abstract**

The present invention relates to an antibody specific for CD47 and uses thereof, and more particularly, to an antibody that specifically binds to CD47, a chimeric antigen receptor comprising the antibody, and a pharmaceutical composition comprising the same for preventing or treating diseases mediated by CD47-expressing cells.

In the present invention, antibodies that more specifically binds to CD47 were screened to establish 7 new types of antibodies (7C7, 5H4, 5A4, 4E12, 3H3, 3A5, 1E7), and it was confirm that the novel antibodies can specifically binds with CD47 antigen.

In addition, since it was confirmed that the production of a chimeric antigen receptor (CAR) targeting CD47 is possible using the established antibody, the CD47-specific antibody of the present invention and the chimeric antigen receptor prepared using the same can be applied for use in preventing or treating a cancer or tumor expressing CD47.

## Description

### [Technical field]

The present invention relates to an antibody specific for CD47 and uses thereof, and more particularly, to an antibody that specifically binds to CD47, a chimeric antigen receptor comprising the antibody, and a pharmaceutical composition for preventing or treating diseases mediated by CD47-expressing cells comprising the same.

### [Background art]

CD47, also called an integrin binding protein (IAP), is a transmembrane glycoprotein widely expressed on the cell surface, and belongs to the immunoglobulin superfamily.

CD47 is a very important marker on the cell surface, with a molecular weight between 47 and 55kD, and has a structure of one amino-terminal extracellular variable region, one transmembrane region composed of three to five highly hydrophobic transmembrane fragments, and one hydrophilic carboxyl terminal cytoplasmic tail. It interacts with various ligands such as integrins, SIRPα (signal regulatory protein α), SIRPγ and thrombospondin.

SIRPα is mainly expressed in bone marrow cells, including macrophages, granulocytes, myeloid dendritic cells (DCs), mast cells, hematopoietic stem cells (HSCs) and their precursors. SIRPα inhibits phagocytosis of host cells by macrophages, and ligation of SIRPα on macrophages by CD47 expressed on host target cells produces a SHP-1 mediated inhibitory signal, thereby negatively regulates to phagocytosing.

In the innate immune system, CD47 functions through binding to SIRPα expressed in myeloid cells, and the action of broad expression of CD47 under physiological conditions prevents healthy cells from being cleared by the innate immune system.

However, tumor cells can effectively evade immune surveillance by overexpression of CD47. In recent years, the CD47 and CD47-SIRPα signaling systems have received the most attention as potential drug targets in tumor therapy. Previous studies have shown that CD47 expression is upregulated and elevated in most human cancers (e.g., NHL, AML, breast cancer, colon cancer, glioblastoma, glioma, ovarian cancer, bladder cancer and prostate cancer). Expression levels of CD47 have been demonstrated to be associated with invasive disease and low survival rates. Weissman of Stanford University systematically studied the expression level of CD47 among various solid tumors. As a result, he found that CD47 was overexpressed in all human solid tumor cells, and the average expression level was 3.3 times that of the corresponding normal cells. In addition, it was found that the level of CD47 mRNA in patients with solid tumors had a negative correlation with the prognostic index (Mark P Chao et al., Front Oncol., 9:1380, 2020).

Anti-CD47 antibody treatment for tumors is associated with various mechanisms. First, the anti-CD47 antibody blocks the binding of CD47 on tumor cells to SIRPα on macrophages, allowing tumor cells to be phagocytosed. In addition, anti-CD47 antibody can induce cytotoxicity of tumor cells involving NK cells, and can eliminate tumor cells by directly inducing apoptosis. Finally, anti-CD47 antibody can activate CD8+ T cells and induce an immune response of acquired T cells to further kill tumor cells.

An antibody specific for CD47 is being developed for the treatment or diagnosis of such CD47-expressing tumor cells or diseases related to CD47 overexpression. International Patent Publication No. WO2018-075857, International Patent Publication No. WO2017-121771 and Publication No. WO2013-119714 discloses various anti-CD47 antibody.

### [Disclosure]

### [Technical Problem]

Therefore, in the present invention, in order to develop an antibody that more specifically binds to CD47, antibodies that can bind to CD47 was screened and 7 new types of antibodies (7C7, 5H4, 5A4, 4E12, 3H3, 3A5, 1E7) were established, and it was confirmed that the 7 kinds of antibody selected in the present invention specifically bound to the CD47 antigen. In addition, it was confirmed that the production of a chimeric antigen receptor targeting CD47 is possible using the CD47-specific antibody of the present invention, and the present invention has been completed.

Accordingly, an object of the present invention is to provide antibodies that specifically binds to CD47.

Another object of the present invention is to provide a polynucleotide encoding the antibody, a vector expressing the antibody, and a recombinant cell transformed with the vector.

Another object of the present invention is to provide a chimeric antigen receptor comprising the antibody, a polynucleotide encoding a chimeric antigen receptor targeting CD47, a vector comprising the same, and an immune effector cell expressing the chimeric antigen receptor comprising the polynucleotide or the vector.

Another object of the present invention is to provide a pharmaceutical composition for preventing or treating a disease mediated by a cell expressing CD47, including the antibody or the immune effector cell expressing the chimeric antigen receptor targeting CD47.

Another object of the present invention is to provide a composition for diagnosing or monitoring a disease mediated by a cell expressing CD47, including the antibody.

### [Technical Solution]

In order to achieve the above object,

The present invention is to provide an antibody specifically binding to CD47 or a fragment thereof, comprising:
(1) a heavy chain variable region including a CDR1 region represented by an amino acid of SEQ ID NO: 1, a CDR2 region represented by an amino acid of SEQ ID NO: 2 and a CDR3 region represented by an amino acid of SEQ ID NO: 3, and a light chain variable region including a CDR1 region represented by an amino acid of SEQ ID NO: 4, a CDR2 region represented by an amino acid of SEQ ID NO: 5 and a CDR3 region represented by an amino acid of SEQ ID NO: 6;
(2) a heavy chain variable region including a CDR1 region represented by an amino acid of SEQ ID NO: 11, a CDR2 region represented by an amino acid of SEQ ID NO: 12 and a CDR3 region represented by an amino acid of SEQ ID NO: 13, and a light chain variable region including a CDR1 region represented by an amino acid of SEQ ID NO: 14, a CDR2 region represented by an amino acid of SEQ ID NO: 15 and a CDR3 region represented by an amino acid of SEQ ID NO: 16;
(3) a heavy chain variable region including a CDR1 region represented by an amino acid of SEQ ID NO: 21, a CDR2 region represented by an amino acid of SEQ ID NO: 22 and a CDR3 region represented by an amino acid of SEQ ID NO: 23, and a light chain variable region including a CDR1 region represented by an amino acid of SEQ ID NO: 24, a CDR2 region represented by an amino acid of SEQ ID NO: 25 and a CDR3 region represented by an amino acid of SEQ ID NO: 26;
(4) a heavy chain variable region including a CDR1 region represented by an amino acid of SEQ ID NO: 31, a CDR2 region represented by an amino acid of SEQ ID NO: 32 and a CDR3 region represented by an amino acid of SEQ ID NO: 33, and a light chain variable region including a CDR1 region represented by an amino acid of SEQ ID NO: 34, a CDR2 region represented by an amino acid of SEQ ID NO: 35 and a CDR3 region represented by an amino acid of SEQ ID NO: 36;
(5) a heavy chain variable region including a CDR1 region represented by an amino acid of SEQ ID NO: 41, a CDR2 region represented by an amino acid of SEQ ID NO: 42 and a CDR3 region represented by an amino acid of SEQ ID NO: 43, and a light chain variable region including a CDR1 region represented by an amino acid of SEQ ID NO: 44, a CDR2 region represented by an amino acid of SEQ ID NO: 45 and a CDR3 region represented by an amino acid of SEQ ID NO: 46;
(6) a heavy chain variable region including a CDR1 region represented by an amino acid of SEQ ID NO: 51, a CDR2 region represented by an amino acid of SEQ ID NO: 52 and a CDR3 region represented by an amino acid of SEQ ID NO: 53, and a light chain variable region including a CDR1 region represented by an amino acid of SEQ ID NO: 54, a CDR2 region represented by an amino acid of SEQ ID NO: 55 and a CDR3 region represented by an amino acid of SEQ ID NO: 56; or
(7) a heavy chain variable region including a CDR1 region represented by an amino acid of SEQ ID NO: 61, a CDR2 region represented by an amino acid of SEQ ID NO: 62 and a CDR3 region represented by an amino acid of SEQ ID NO: 63, and a light chain variable region including a CDR1 region represented by an amino acid of SEQ ID NO: 64, a CDR2 region represented by an amino acid of SEQ ID NO: 65 and a CDR3 region represented by an amino acid of SEQ ID NO: 66.

In a preferred embodiment of the present invention, the antibody may be a monoclonal antibody, preferably a single-chain variable fragment (scFv).

In another preferred embodiment of the present invention, the (1) antibody may comprise a heavy chain variable region represented by an amino acid of SEQ ID NO: 7 and a light chain variable region represented by an amino acid of SEQ ID NO: 8;
the (2) antibody may comprise a heavy chain variable region represented by an amino acid of SEQ ID NO: 17 and a light chain variable region represented by an amino acid of SEQ ID NO: 18;
the (3) antibody may comprise a heavy chain variable region represented by an amino acid of SEQ ID NO: 27 and a light chain variable region represented by an amino acid of SEQ ID NO: 28;
the (4) antibody may comprise a heavy chain variable region represented by an amino acid of SEQ ID NO: 37 and a light chain variable region represented by an amino acid of SEQ ID NO: 38;
the (5) antibody may comprise a heavy chain variable region represented by an amino acid of SEQ ID NO: 47 and a light chain variable region represented by an amino acid of SEQ ID NO: 48;
the (6) antibody may comprise a heavy chain variable region represented by an amino acid of SEQ ID NO: 57 and a light chain variable region represented by an amino acid of SEQ ID NO: 58; or
the (7) antibody may comprise a heavy chain variable region represented by an amino acid of SEQ ID NO: 67 and a light chain variable region represented by an amino acid of SEQ ID NO: 68.

In another preferred embodiment of the present invention, the antibody can prevent CD47 from interacting with signal-regulating-protein α (SIRPα) or promote macrophage-mediated phagocytosis on CD47-expressing cells.

To achieve another object, the present invention provides a polynucleotide encoding the antibody that specifically binds to CD47.

In addition, the present invention provides a vector comprising a polynucleotide encoding the antibody that specifically binds to CD47.

In addition, the present invention provides a recombinant cell transformed with the vector that produces the antibody or a fragment thereof that specifically binds to CD47.

In order to achieve another object, the present invention provides a chimeric antigen receptor (CAR) comprising: a CD47-binding domain; a transmembrane domain; a costimulatory domain; and an intracellular signal transduction domain, wherein the CD47-binding domain may be selected from the antibody specifically binding to CD47 or a fragment thereof.

The CD47-binding domain may be selected from the antibody or a fragment thereof capable of specifically binding to CD47 of the present invention.

In a preferred embodiment of the present invention, the transmembrane domain may be derived from a protein selected from the group consisting of CD8α, CD4, CD28, CD137, CD80, CD86, CD152 and PD1.

In another preferred embodiment of the present invention, the costimulatory domain may be derived from a protein selected from the group consisting of CD28, 4-1BB, OX-40 and ICOS, and the signaling domain may be derived from CD3ζ.

In another preferred embodiment of the present invention, a hinge region located between the C terminus of the CD47-binding domain and the N terminus of the transmembrane domain may be further included, wherein the hinge region may be derived from CD8α.

In order to achieve another object, the present invention provides a polynucleotide encoding the chimeric antigen receptor (CAR).

In addition, the present invention provides a vector comprising a polynucleotide encoding a chimeric antigen receptor (CAR).

In a preferred embodiment of the present invention, the vector may be a plasmid, a retroviral vector, or a lentiviral vector.

In addition, the present invention provides an immune effector cell expressing the chimeric antigen receptor (CAR) comprising the polynucleotide encoding the chimeric antigen receptor.

In a preferred embodiment of the present invention, the immune effector cell may be a T cell.

In order to achieve another object, the present invention provides a pharmaceutical composition for use in preventing or treating a cancer or tumor expressing CD47, comprising the antibody specifically binding to CD47 or the fragment thereof of the invention or the immune effector cell expressing a chimeric antigen receptor targeting CD47 of the invention.

In the present invention, the cancer or tumor may be selected from the group consisting of hematologic cancer (blood cancer), ovarian cancer, colon cancer, breast cancer, lung cancer, myeloma, neuroblast-derived CNS cancer, monocytic leukemia, B-cell leukemia, T-cell leukemia, B-cell lymphoma, T-cell lymphoma, and mast cell-derived cancer.

### [Advantageous Effects]

In the present invention, antibodies that more specifically bind to CD47 were screened to establish 7 new types of antibodies (7C7, 5H4, 5A4, 4E12, 3H3, 3A5, 1E7), and the novel antibodies specifically combined with the CD47 antigen were confirmed.

In addition, since it was confirmed that the production of a chimeric antigen receptor (CAR) targeting CD47 is possible using the established antibody, the CD47-specific antibody of the present invention and the chimeric antigen receptor prepared using the same can be applied to the prevention or treatment of cancers or tumors expressing CD47.

### [Description of Drawings]

Fig. 1 is data confirming the binding ability of 7C7, 5H4, 5A4, 4E12, 3H3, 3A5 and 1E7 antibodies selected in the present invention to CD47-expressing tumor cells (MCF-7) by flow cytometry.
Fig. 2 is a schematic diagram illustrating a method for preparing CD47-CAR-expressing cells using the lentivirus expressing a chimeric antigen receptor (CD47-CAR) targeting CD47.
Fig. 3 is a schematic diagram illustrating a method of confirming the binding ability of the transformed HEK293 cells to the CD47 peptide after transforming the HEK293 cell line with a CD47-CAR expression lentivirus vector.
Fig. 4 is data confirming CD47-CAR expression and binding ability to CD47 in HEK293FT cells transformed with a lentivirus vector expressing CD47-CAR.

### [Mode of the Invention]

Hereinafter, the present invention is described in detail.

### Antibody that specifically binds to CD47

The present invention relates to an antibody specifically binding to CD47 or a fragment thereof, comprising:
(1) a heavy chain variable region including a CDR1 region represented by an amino acid of SEQ ID NO: 1, a CDR2 region represented by an amino acid of SEQ ID NO: 2 and a CDR3 region represented by an amino acid of SEQ ID NO: 3, and a light chain variable region including a CDR1 region represented by an amino acid of SEQ ID NO: 4, a CDR2 region represented by an amino acid of SEQ ID NO: 5 and a CDR3 region represented by an amino acid of SEQ ID NO: 6;
(2) a heavy chain variable region including a CDR1 region represented by an amino acid of SEQ ID NO: 11, a CDR2 region represented by an amino acid of SEQ ID NO: 12 and a CDR3 region represented by an amino acid of SEQ ID NO: 13, and a light chain variable region including a CDR1 region represented by an amino acid of SEQ ID NO: 14, a CDR2 region represented by an amino acid of SEQ ID NO: 15 and a CDR3 region represented by an amino acid of SEQ ID NO: 16;
(3) a heavy chain variable region including a CDR1 region represented by an amino acid of SEQ ID NO: 21, a CDR2 region represented by an amino acid of SEQ ID NO: 22 and a CDR3 region represented by an amino acid of SEQ ID NO: 23, and a light chain variable region including a CDR1 region represented by an amino acid of SEQ ID NO: 24, a CDR2 region represented by an amino acid of SEQ ID NO: 25 and a CDR3 region represented by an amino acid of SEQ ID NO: 26;
(4) a heavy chain variable region including a CDR1 region represented by an amino acid of SEQ ID NO: 31, a CDR2 region represented by an amino acid of SEQ ID NO: 32 and a CDR3 region represented by an amino acid of SEQ ID NO: 33, and a light chain variable region including a CDR1 region represented by an amino acid of SEQ ID NO: 34, a CDR2 region represented by an amino acid of SEQ ID NO: 35 and a CDR3 region represented by an amino acid of SEQ ID NO: 36;
(5) a heavy chain variable region including a CDR1 region represented by an amino acid of SEQ ID NO: 41, a CDR2 region represented by an amino acid of SEQ ID NO: 42 and a CDR3 region represented by an amino acid of SEQ ID NO: 43, and a light chain variable region including a CDR1 region represented by an amino acid of SEQ ID NO: 44, a CDR2 region represented by an amino acid of SEQ ID NO: 45 and a CDR3 region represented by an amino acid of SEQ ID NO: 46;
(6) a heavy chain variable region including a CDR1 region represented by an amino acid of SEQ ID NO: 51, a CDR2 region represented by an amino acid of SEQ ID NO: 52 and a CDR3 region represented by an amino acid of SEQ ID NO: 53, and a light chain variable region including a CDR1 region represented by an amino acid of SEQ ID NO: 54, a CDR2 region represented by an amino acid of SEQ ID NO: 55 and a CDR3 region represented by an amino acid of SEQ ID NO: 56; or
(7) a heavy chain variable region including a CDR1 region represented by an amino acid of SEQ ID NO: 61, a CDR2 region represented by an amino acid of SEQ ID NO: 62 and a CDR3 region represented by an amino acid of SEQ ID NO: 63, and a light chain variable region including a CDR1 region represented by an amino acid of SEQ ID NO: 64, a CDR2 region represented by an amino acid of SEQ ID NO: 65 and a CDR3 region represented by an amino acid of SEQ ID NO: 66.

In the present invention, the antibody can prevent CD47 from interacting with signal-regulating-protein α (SIRPα) or promote macrophage-mediated phagocytosis on CD47-expressing cells.

In the present invention, the antibody may be a monoclonal antibody. In the present invention, the term "monoclonal antibody" may be an antibody produced by a single antibody-forming cell, with a uniform primary structure (amino acid sequence). It recognizes only one antigenic determinant and is generally produced by culturing a hybridoma cell in which cancer cells and an antibody-producing cell are fused.

The antibody of the present invention is prepared as a humanized antibody with increased similarity to a human antibody by making the remaining parts except for the CDR region, which is a key part for antigen binding, to an amino acid sequence corresponding to an antibody produced by humans. The most common method for humanizing antibody is a CDR-grafting method in which the CDR regions of an animal antibody are grafted into a human antibody, but is not limited thereto, and is known in the art.

As used herein, the term "CDR (complementarity determining region)", refers to a non-contiguous antigen binding site found within the variable region of both heavy and light chain polypeptides.

In the present invention, the term "antibody" can be used not only in a complete form having two full-length light chains and two full-length heavy chains, but also fragments of antibody molecule. A fragment of antibody molecule means a fragment having at least a peptide tag (epitope) binding function, and includes scFv, Fab, F(ab'), F(ab')₂, a single domain, etc.

Among antibody fragments, Fab has a structure having variable regions of light and heavy chains, a constant region of light chain and the first constant region of heavy chain (CH1), and has one antigen-binding site. Fab' differs from Fab in that it has a hinge region comprising one or more cysteine residues at the C terminus of the heavy chain CH1 domain. F(ab')₂ antibody is produced by forming a disulfide bond with a cysteine residue in the hinge region of Fab'. Fv is a minimal antibody fragment having only a heavy chain variable region and a heavy chain variable region. Recombinant technology for generating an Fv fragment is described in International Patent Publications WO 88/10649, WO 88/106630, WO 88/07085, WO 88 /07086 and WO 88/09344. A double chain Fv (dsFv) has a disulfide bond, and a heavy chain variable region and a heavy chain variable region are connected, and a single chain Fv (scFv) is generally connected through a peptide linker, the variable region of the heavy chain and the variable region of the light chain are covalently bonded. Such an antibody fragment can be obtained using a proteolytic enzyme (for example, Fab can be obtained by restriction digestion of the entire antibody with papain, and F(ab')₂ fragment can be obtained by digestion with pepsin). Preferably, it can be produced through genetic recombination technology.

The monoclonal antibody that specifically binds to CD47 of the present invention can be prepared by using all or part of the CD47 protein as an immunogen (or antigen). More specifically, as an immunogen, CD47, a fusion protein containing CD47 protein, or a carrier containing CD47 protein, if necessary, together with an adjuvant (e.g., Freund adjuvant), is injected once or more by subcutaneous, intramuscular, intravenous, intraperitoneal in mammals except for humans to achieve an immunization. The mammals other than humans are preferably mice, rats, hamsters, malmots, chickens, rabbits, cats, dogs, pigs, goats, sheep, donkeys, horses or cattle (including transgenic animals engineered to produce an antibody from other animals such as mice to produce human antibody), more preferably mouse, rat, hamster, malmot, chicken or rabbit. Antibody-producing cells can be obtained from the immune-sensitized mammal about 1 to 10 days after the final immunization by performing immunization 1 to 4 times every 1 to 21 days from the first immunization. The number of times and intervals for immunization can be appropriately changed depending on the characteristics of the immunogen to be used.

Preparation of a hybridoma secreting a monoclonal antibody can be carried out according to the method of Keira and Mirstein et al. (Nature, 1975, Vol. 256, p. 495-497) and a method similar thereto. Hybridomas can be produced by cell fusion of mammal-derived myeloma cells without autologous antibody-producing ability and antibody-producing cells contained in the group consisting of spleen, lymph node, bone marrow and tonsils, preferably spleen. The mammal may be a mouse, rat, malmot, hamster, chicken, rabbit or human, preferably a mouse, rat, chicken or human.

For cell fusion, for example, a fusion promoter including polyethylene glycol or Sendai virus or a method by electric pulse is used, for example, in a fusion medium containing a fusion promoter, antibody-producing cells and mammalian-derived cells capable of indefinite proliferation. Cells are suspended at a ratio of about 1:1 to 1:10, and in this state, cultured at about 30 to 40°C for about 1 to 5 minutes. As the fusion medium, for example, MEM medium, RPMI1640 medium, and Iscove's Modified Dulbecco's Medium may be used, and it is preferable to exclude sera such as bovine serum.

In the method of screening the hybridoma clones producing the monoclonal antibody, first, the fusion cells obtained as described above are transferred to a selection medium such as HAT medium, and cultured at about 30 to 40°C. for about 3 days to 3 weeks to kill cells other than hybridomas. Then, after culturing the hybridoma on a microtiter plate, etc., the part with increased reactivity between the immunogen used for the immune response of animals other than humans described above and the culture supernatant was subjected to RIA (radioactive substance-marked immuno antibody) or ELISA (Enzyme-Linked Immunosorbent Assay). The clone producing the monoclonal antibody found above shows specific binding ability to the immunogen.

The monoclonal antibody of the present invention can be obtained by culturing such a hybridoma in vitro or in vivo. For culturing, a conventional method for culturing cells derived from mammals is used, and for collecting monoclonal antibody from a culture or the like, a conventional method in this field for purifying an antibody in general is used. As each method, for example, salting out, dialysis, filtration, concentration, centrifugation, fractional precipitation, gel filtration chromatography, ion exchange chromatography, affinity chromatography, high-performance liquid chromatography, gel electrophoresis or isoelectric point electrophoresis, etc. can be applied, and these are applied in combination as needed. The purified monoclonal antibody is then concentrated and dried to be in a liquid or solid state depending on the use.

In a specific embodiment of the present invention, in order to prepare the antibody that specifically binds to CD47, hybridomas that produce CD47 protein are prepared and screened, and 7 kinds of antibodies (scFvs) that specifically bind to CD47 were selected and designated as 7C7, 5H4, 5A4, 4E12, 3H3, 3A5 and 1E7, respectively.
(1) It was confirmed that 7C7 antibody had a heavy chain variable region including a CDR1 region represented by an amino acid of SEQ ID NO: 1 (GYTFTSYV), a CDR2 region represented by an amino acid of SEQ ID NO: 2 (INPYNDGT) and a CDR3 region represented by an amino acid of SEQ ID NO: 3 (ARGRNRYDSWFAY), and a light chain variable region including a CDR1 region represented by an amino acid of SEQ ID NO: 4 (QDISNY), a CDR2 region represented by an amino acid of SEQ ID NO: 5 (YTS) and a CDR3 region represented by an amino acid of SEQ ID NO: 6 (QQGNTLPWT).

Specifically, 7C7 antibody contained a heavy chain variable region represented by the amino acid of SEQ ID NO: 7 and a light chain variable region represented by the amino acid of SEQ ID NO: 8, wherein the heavy chain variable region was encoded with the nucleotide sequence of SEQ ID NO: 9 and a light chain variable region was encoded with the nucleotide sequence of SEQ ID NO: 10.

(2) It was confirmed that 5H4 antibody had a heavy chain variable region including a CDR1 region represented by an amino acid of SEQ ID NO: 11 (GYTFTNYW), a CDR2 region represented by an amino acid of SEQ ID NO: 12 (IDPSNSAT) and a CDR3 region represented by an amino acid of SEQ ID NO: 13 (ARGGFAFDS), and a light chain variable region including a CDR1 region represented by an amino acid of SEQ ID NO: 14 (QSLVHSNGNTY), a CDR2 region represented by an amino acid of SEQ ID NO: 15 (KVS) and a CDR3 region represented by an amino acid of SEQ ID NO: 16 (SQSTHVPWT).

Specifically, 5H4 antibody contained a heavy chain variable region represented by the amino acid of SEQ ID NO: 17 and a light chain variable region represented by the amino acid of SEQ ID NO: 18, wherein the heavy chain variable region was encoded with the nucleotide sequence of SEQ ID NO: 19 and a light chain variable region was encoded with the nucleotide sequence of SEQ ID NO: 20.

(3) It was confirmed that 5A4 antibody had a heavy chain variable region including a CDR1 region represented by an amino acid of SEQ ID NO: 21 (GYTFTNYW), a CDR2 region represented by an amino acid of SEQ ID NO: 22 (IDPSDSYT) and a CDR3 region represented by an amino acid of SEQ ID NO: 23 (TRGGKRAMDY), and a light chain variable region including a CDR1 region represented by an amino acid of SEQ ID NO: 24 (QSLVHSNGNTY), a CDR2 region represented by an amino acid of SEQ ID NO: 25 (KVS) and a CDR3 region represented by an amino acid of SEQ ID NO: 26 (SQSTHVPFT).

Specifically, 5A4 antibody contained a heavy chain variable region represented by the amino acid of SEQ ID NO: 27 and a light chain variable region represented by the amino acid of SEQ ID NO: 28, wherein the heavy chain variable region was encoded with the nucleotide sequence of SEQ ID NO: 29 and a light chain variable region was encoded with the nucleotide sequence of SEQ ID NO: 30.

(4) It was confirmed that 4E12 antibody had a heavy chain variable region including a CDR1 region represented by an amino acid of SEQ ID NO: 31 (GYTFTNYG), a CDR2 region represented by an amino acid of SEQ ID NO: 32 (INTYTGEP) and a CDR3 region represented by an amino acid of SEQ ID NO: 33 (ARGGGRGAMDY), and a light chain variable region including a CDR1 region represented by an amino acid of SEQ ID NO: 34 (QSIVHSNGNTY), a CDR2 region represented by an amino acid of SEQ ID NO: 35 (KVS) and a CDR3 region represented by an amino acid of SEQ ID NO: 36 (FQGSHVPFT).

Specifically, 4E12 antibody contained a heavy chain variable region represented by the amino acid of SEQ ID NO: 37 and a light chain variable region represented by the amino acid of SEQ ID NO: 38, wherein the heavy chain variable region was encoded with the nucleotide sequence of SEQ ID NO: 39 and a light chain variable region was encoded with the nucleotide sequence of SEQ ID NO: 40.

(5) It was confirmed that 3H3 antibody had a heavy chain variable region including a CDR1 region represented by an amino acid of SEQ ID NO: 41 (GYTFTNYW), a CDR2 region represented by an amino acid of SEQ ID NO: 42 (IDPSNSET) and a CDR3 region represented by an amino acid of SEQ ID NO: 43 (ARGGFAFDS), and a light chain variable region including a CDR1 region represented by an amino acid of SEQ ID NO: 44 (QSLVHNNGNTY), a CDR2 region represented by an amino acid of SEQ ID NO: 45 (KVS) and a CDR3 region represented by an amino acid of SEQ ID NO: 46 (SQSTHVPWT).

Specifically, 3H3 antibody contained a heavy chain variable region represented by the amino acid of SEQ ID NO: 47 and a light chain variable region represented by the amino acid of SEQ ID NO: 48, wherein the heavy chain variable region was encoded with the nucleotide sequence of SEQ ID NO: 49 and a light chain variable region was encoded with the nucleotide sequence of SEQ ID NO: 50.

(6) It was confirmed that 3A5 antibody had a heavy chain variable region including a CDR1 region represented by an amino acid of SEQ ID NO: 51 (GYTFTSYW), a CDR2 region represented by an amino acid of SEQ ID NO: 52 (IDPSDSYT) and a CDR3 region represented by an amino acid of SEQ ID NO: 53 (ARGGKRAMDY), and a light chain variable region including a CDR1 region represented by an amino acid of SEQ ID NO: 54 (QSLVHSNGNTY), a CDR2 region represented by an amino acid of SEQ ID NO: 55 (KVS) and a CDR3 region represented by an amino acid of SEQ ID NO: 56 (SQSTHVPFT).

Specifically, 3A5 antibody contained a heavy chain variable region represented by the amino acid of SEQ ID NO: 57 and a light chain variable region represented by the amino acid of SEQ ID NO: 58, wherein the heavy chain variable region was encoded with the nucleotide sequence of SEQ ID NO: 59 and a light chain variable region was encoded with the nucleotide sequence of SEQ ID NO: 60.

(7) It was confirmed that 1E7 antibody had a heavy chain variable region including a CDR1 region represented by an amino acid of SEQ ID NO: 61 (GYIFTSYV), a CDR2 region represented by an amino acid of SEQ ID NO: 62 (INPYNDGT) and a CDR3 region represented by an amino acid of SEQ ID NO: 63 (ARGGFTTDY), and a light chain variable region including a CDR1 region represented by an amino acid of SEQ ID NO: 64 (QSLVHSNGNTY), a CDR2 region represented by an amino acid of SEQ ID NO: 65 (KVS) and a CDR3 region represented by an amino acid of SEQ ID NO: 66 (SQSTHVPYT).

Specifically, 1E7 antibody contained a heavy chain variable region represented by the amino acid of SEQ ID NO: 67 and a light chain variable region represented by the amino acid of SEQ ID NO: 68, wherein the heavy chain variable region was encoded with the nucleotide sequence of SEQ ID NO: 69 and a light chain variable region was encoded with the nucleotide sequence of SEQ ID NO: 70.

The antibody specific for CD47 of the present invention is preferably scFv (single chain variable fragment), and can be produced through genetic recombination technology so that the heavy chain variable region and a light chain variable region can be linked with a linker. The linker may preferably be represented by the amino acid sequence of SEQ ID NO: 71 or the nucleotide sequence of SEQ ID NO: 72, but is not limited thereto.

When linked by a light chain variable region-linker-a heavy chain variable region, 7C7 antibody has the amino acid sequence of SEQ ID NO: 73 or the nucleotide sequence of SEQ ID NO: 74, and 5H4 antibody has the amino acid sequence of SEQ ID NO: 75 or the nucleotide sequence of SEQ ID NO: 76, 5A4 antibody has the amino acid sequence of SEQ ID NO: 77 or the nucleotide sequence of SEQ ID NO: 78, and 4E12 antibody has the amino acid sequence of SEQ ID NO: 79 or the nucleotide sequence of SEQ ID NO: 80, 3H3 antibody has the amino acid sequence of SEQ ID NO: 81 or the nucleotide sequence of SEQ ID NO: 82, 3A5 antibody has the amino acid sequence of SEQ ID NO: 83 or the nucleotide sequence of SEQ ID NO: 84, and 1E7 antibody has the amino acid sequence of SEQ ID NO: 85 or the nucleotide sequence of SEQ ID NO: 86.

In another aspect, the present invention relates to a polynucleotide encoding the antibody that specifically binds to CD47.

As used herein, the term "polynucleotide" generally refers to a nucleic acid molecule, deoxyribonucleotide or ribonucleotide, or an analog thereof, separated by any length. In some embodiments, a polynucleotide of the present invention can be prepared by (1) in-vitro amplification, such as polymerase chain reaction (PCR) amplification; (2) cloning and recombination; (3) purification such as digestion and gel electrophoretic separation; (4) synthesis such as chemical synthesis, and preferably, the isolated polynucleotide is prepared by recombinant DNA technology. In the present invention, the nucleic acid for encoding the antibody or antigen-binding fragment thereof can be prepared by various methods known in the art, including, but not limited to, restriction fragment operation of synthetic oligonucleotides or application of SOE PCR.

In another aspect, the present invention relates to a vector comprising the polynucleotide encoding the antibody that specifically binds to CD47, and a recombinant cell transformed with the vector.

In the present invention, the term "vector (expression vector)" refers to a gene preparation including essential regulatory elements such as a promoter so that a target gene can be expressed in an appropriate host cell. A vector may be selected from one or more of a plasmid, a retroviral vector, and a lentiviral vector. Upon transformation into an appropriate host, a vector can replicate and function independently of the host genome, or in some cases can be integrated into the genome itself.

In addition, a vector may contain expression control elements that allow the coding region to be accurately expressed in a suitable host. Such regulatory elements are well known to those skilled in the art and include, for example, promoters, ribosome-binding sites, enhancers and other regulatory elements for regulating gene transcription or mRNA translation. The specific structure of the expression control sequence may vary depending on the function of the species or cell type, but generally contains 5' non-translated sequence, and a 5' or 3' non-translated sequence participating in transcription initiation and translation initiation, respectively, such as TATA box, capped sequence, CAAT sequence, etc. For example, a 5' non-transcriptional expression control sequence can include a promoter region that can include a promoter sequence for transcription and control of a functionally linked nucleic acid.

As used herein, the term "promoter" means a minimal sequence sufficient to direct transcription. In addition, promoter constructs sufficient to allow expression of a regulatable promoter-dependent gene induced by cell type-specific or external signals or agents may be included, and these constructs may be located in the 5' or 3' portion of the gene. Both conservative and inducible promoters are included. Promoter sequences may be derived from prokaryotes, eukaryotes or viruses.

In the present invention, the term "transformant" refers to a cell transformed by introducing a vector having a polynucleotide encoding one or more target proteins into a host cell, and a method for introducing the expression a vector into the host cell to form a transformant are such as a calcium phosphate method or a calcium chloride/rubidium chloride method, an electroporation method, an electroinjection method, a chemical treatment method such as PEG, a method using a gene gun, and the like (Sambrook, J., et al., Molecular Cloning, A Laboratory Manual(2nd ed.), Cold Spring Harbor Laboratory, 1. 74, 1989).

When the transformant expressing the vector is cultured in a nutrient medium, an antibody protein can be produced and isolated in large quantities. Medium and culture conditions can be appropriately selected and used depending on the host cell. During culture, conditions such as temperature, medium pH, and culture time should be appropriately adjusted to be suitable for cell growth and mass production of proteins.

The vector according to the present invention can be transformed into a host cell, preferably a mammalian cell, for the production of the antibody. Suitable host cells capable of expressing fully glycosylated proteins include COS-1 (e.g., ATCC CRL 1650), COS-7 (e.g., ATCC CRL-1651), HEK293, BHK21 (e.g., ATCC CRL-10), CHO (e.g., ATCC CRL 1610) and BSC-1 (e.g., ATCC CRL-26) cell lines, Cos-7 cells, CHO cells, hep G2 cells, P3X63Ag8.653, SP2/0 -Agl4, 293 cells, HeLa cells, etc., and these cells are readily available from, for example, ATCC (American Type Culture Collection, USA).

### Chimeric antigen receptor targeting CD47

The present invention also relates to a chimeric antigen receptor (CAR) comprising: a CD47-binding domain; a transmembrane domain; a costimulatory domain; and an intracellular signal transduction domain,
wherein the CD47-binding domain is an antibody specifically binding to CD47 or a fragment thereof, comprising:
(1) a heavy chain variable region including a CDR1 region represented by an amino acid of SEQ ID NO: 1, a CDR2 region represented by an amino acid of SEQ ID NO: 2 and a CDR3 region represented by an amino acid of SEQ ID NO: 3, and a light chain variable region including a CDR1 region represented by an amino acid of SEQ ID NO: 4, a CDR2 region represented by an amino acid of SEQ ID NO: 5 and a CDR3 region represented by an amino acid of SEQ ID NO: 6;
(2) a heavy chain variable region including a CDR1 region represented by an amino acid of SEQ ID NO: 11, a CDR2 region represented by an amino acid of SEQ ID NO: 12 and a CDR3 region represented by an amino acid of SEQ ID NO: 13, and a light chain variable region including a CDR1 region represented by an amino acid of SEQ ID NO: 14, a CDR2 region represented by an amino acid of SEQ ID NO: 15 and a CDR3 region represented by an amino acid of SEQ ID NO: 16;
(3) a heavy chain variable region including a CDR1 region represented by an amino acid of SEQ ID NO: 21, a CDR2 region represented by an amino acid of SEQ ID NO: 22 and a CDR3 region represented by an amino acid of SEQ ID NO: 23, and a light chain variable region including a CDR1 region represented by an amino acid of SEQ ID NO: 24, a CDR2 region represented by an amino acid of SEQ ID NO: 25 and a CDR3 region represented by an amino acid of SEQ ID NO: 26;
(4) a heavy chain variable region including a CDR1 region represented by an amino acid of SEQ ID NO: 31, a CDR2 region represented by an amino acid of SEQ ID NO: 32 and a CDR3 region represented by an amino acid of SEQ ID NO: 33, and a light chain variable region including a CDR1 region represented by an amino acid of SEQ ID NO: 34, a CDR2 region represented by an amino acid of SEQ ID NO: 35 and a CDR3 region represented by an amino acid of SEQ ID NO: 36;
(5) a heavy chain variable region including a CDR1 region represented by an amino acid of SEQ ID NO: 41, a CDR2 region represented by an amino acid of SEQ ID NO: 42 and a CDR3 region represented by an amino acid of SEQ ID NO: 43, and a light chain variable region including a CDR1 region represented by an amino acid of SEQ ID NO: 44, a CDR2 region represented by an amino acid of SEQ ID NO: 45 and a CDR3 region represented by an amino acid of SEQ ID NO: 46;
(6) a heavy chain variable region including a CDR1 region represented by an amino acid of SEQ ID NO: 51, a CDR2 region represented by an amino acid of SEQ ID NO: 52 and a CDR3 region represented by an amino acid of SEQ ID NO: 53, and a light chain variable region including a CDR1 region represented by an amino acid of SEQ ID NO: 54, a CDR2 region represented by an amino acid of SEQ ID NO: 55 and a CDR3 region represented by an amino acid of SEQ ID NO: 56; or
(7) a heavy chain variable region including a CDR1 region represented by an amino acid of SEQ ID NO: 61, a CDR2 region represented by an amino acid of SEQ ID NO: 62 and a CDR3 region represented by an amino acid of SEQ ID NO: 63, and a light chain variable region including a CDR1 region represented by an amino acid of SEQ ID NO: 64, a CDR2 region represented by an amino acid of SEQ ID NO: 65 and a CDR3 region represented by an amino acid of SEQ ID NO: 66.

As used herein, the term "chimeric antigen receptor (CAR)" generally refers to a fusion protein containing an extracellular domain having the ability to bind an antigen and one or more intracellular domains. A CAR is a core part of a chimeric antigen receptor T cell (CAR-T) and may contain an antigen binding domain, a transmembrane domain, a co-stimulatory domain, and an intracellular signal transduction domain. A CAR can be combined with a T cell receptor-activating intracellular domain based on the antigen (e.g., CD47) specificity of the antibody. Genetically modified CAR-expressing T cells can specifically identify and eliminate target antigen-expressing malignant cells.

In the present invention, the term "CD47-binding domain" generally refers to a domain capable of specifically binding to a CD47 protein. For example, the CD47-binding domain may contain an anti-CD47 antibody or a fragment thereof capable of specifically binding to a human CD47 polypeptide or a fragment thereof expressed in B cells.

In the present invention, the term "binding domain " can be used interchangeably refers to "extracellular domain", "extracellular binding domain", "antigen-specific binding domain" and "extracellular antigen-specific binding domain" and refers to a CAR domain or fragment that has the ability to specifically bind to a target antigen (e.g., CD47).

In the present invention, anti-CD47 antibody or a fragment thereof is the aforementioned anti-CD47 antibody, a monoclonal antibody, preferably a single chain variable fragment (scFv). Specifically, it can be prepared using 7C7, 5H4, 5A4, 4E12, 3H3, 3A5 and 1E7 antibody specific for CD47 of the present invention.

In the present invention, a signal peptide may be further included at the N-terminus of the CD47-binding domain, and the "signal peptide" generally refers to a peptide chain for guiding protein transduction. The signal peptide may be a short peptide having a length of 5 to 30 amino acids, preferably represented by the amino acid sequence of SEQ ID NO: 94.

In the present invention, it may further include a hinge region located between the C terminus of the CD47-binding domain and the N terminus of a transmembrane domain, wherein the hinge region is derived from CD8α, and preferably, it can be represented by the amino acid sequence of SEQ ID NO: 95. The "hinge region" generally refers to the linking region between an antigen-binding region and an immune cell Fc receptor (FcR)-binding region.

In the present invention, "a transmembrane domain" refers to a domain of a CAR that generally passes through a cell membrane and is connected to an intracellular signal transduction domain to play a role in signal transduction. The transmembrane domain may be derived from a protein selected from the group consisting of CD8α, CD4, CD28, CD137, CD80, CD86, CD152 and PD1, and preferably may be represented by the amino acid sequence of SEQ ID NO: 96.

In the present invention, "costimulatory domain" generally refers to an intracellular domain capable of providing immune-stimulatory molecules, which are cell surface molecules necessary for an effective response of lymphocytes to antigens. The costimulatory domain described above may comprise a costimulatory domain of CD28, and may comprise a costimulatory domain of the TNF receptor family, such as the costimulatory domain of OX40 and 4-1BB, preferably it may be 4-1BB represented by the amino acid sequence of SEQ ID NO: 97.

In the present invention, "intracellular signal transduction domain" generally refers to a domain located inside a cell and capable of transmitting a signal. In the present invention, the intracellular signal transduction domain is an intracellular signal transduction domain of the chimeric antigen receptor. For example, the intracellular signal transduction domain may be selected from CD3ζ intracellular domain, CD28 intracellular domain, CD28 intracellular domain, 4-1BB intracellular domain and OX40 intracellular domain, and preferably it may be CD3ζ represented by the amino acid sequence of SEQ ID NO: 98.

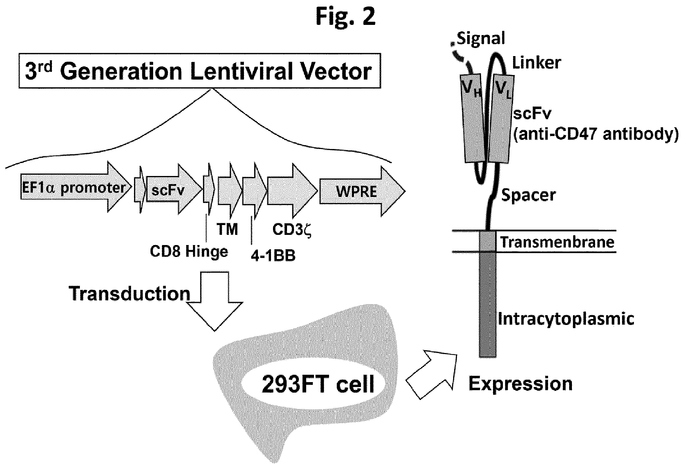

The chimeric antigen receptor targeting CD47 of the present invention (CD47-CAR) can be preferably prepared as shown in the schematic diagram shown in FIG. 2.

### Polynucleotide coding chimeric antigen receptor and Vector expressing chimeric antigen receptor

In another aspect, the present invention relates to a polynucleotide encoding the chimeric antigen receptor (CAR).

In the present invention, the polynucleotide encoding a chimeric antigen receptor (CAR) is a polynucleotide encoding a CD47-binding domain; a polynucleotide encoding a transmembrane domain; a polynucleotide encoding a costimulatory domain; and a polynucleotide encoding an intracellular signal transduction domain.

A polynucleotide encoding the CD47-binding domain may be a polynucleotide encoding the antibody specific for CD47 of the present invention, 7C7, 5H4, 5A4, 4E12, 3H3, 3A5 and/or 1E7, and a light chain variable region and a heavy chain variable region may be in the form of scFv linked by a linker, and the specific nucleotide sequence may be the same as described above.

Preferably, a polynucleotide encoding a chimeric antigen receptor (CAR) of the present invention may has:
a signal peptide represented by the nucleotide sequence of SEQ ID NO: 88;
7C7 antibody represented by the nucleotide sequence of SEQ ID NO: 74, 5H4 antibody represented by the nucleotide sequence of SEQ ID NO: 76, 5A4 antibody represented by the nucleotide sequence of SEQ ID NO: 78, 4E12 antibody represented by the nucleotide sequence of SEQ ID NO: SEQ ID NO: 80, 3H3 antibody represented by the nucleotide sequence of SEQ ID NO: 82, 3A5 antibody represented by the nucleotide sequence of SEQ ID NO: 84, or 1E7 antibody represented by the nucleotide sequence of SEQ ID NO: 86;
a transmembrane domain represented by the nucleotide sequence of 90;
4-1BB (a costimulatory domain) represented by the nucleotide sequence of SEQ ID NO: 91; and
an intracellular signal transduction domain (CD3ζ) represented by the nucleotide sequence of SEQ ID NO: 92.

In addition, a polynucleotide encoding a hinge region may be additionally included between a polynucleotide encoding the CD47-binding domain and a transmembrane domain, and preferably It may be a CD8 hinge region represented by the nucleotide sequence of SEQ ID NO: 89.

In another aspect, the present invention relates to a vector comprising a polynucleotide encoding the chimeric antigen receptor (CAR).

In a specific embodiment of the present invention, the vector is a recombinant virus a vector, preferably a lentivirus vector, and comprises an operably linked EF1α promoter; a polynucleotide encoding a signal peptide; a polynucleotide encoding a CD47-binding domain; a polynucleotide encoding a transmembrane domain; and a polynucleotide encoding an intracellular signal transduction domain, and may further include a woodchuck hepatitis virus post-transcriptional regulatory element (WPRE) to increase protein expression (refer to FIG. 2).

The EF1α promoter may be represented by the nucleotide sequence of SEQ ID NO: 87, and if necessary, has 90% or more, 93% or more, 95% or more, 96% or more, 97% or more, 98% or more, or 99% or more identical sequences of the nucleotide sequence of SEQ ID NO: 87.

In addition, the promoter is operably linked to induce expression of an anti-CD47 antibody (scFv), which is a CD47-binding domain.

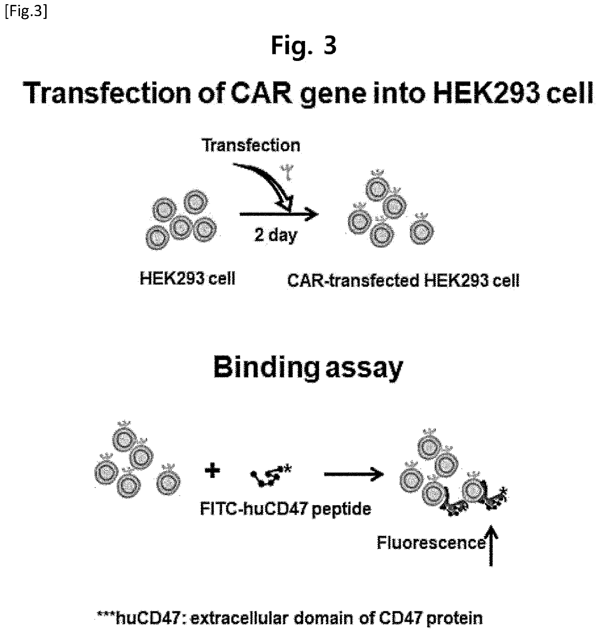

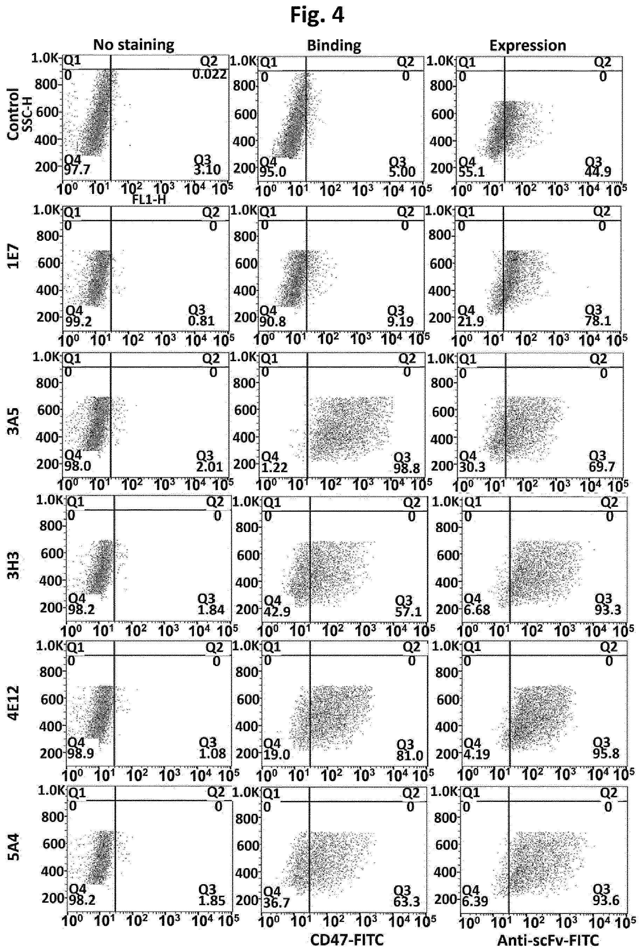

In a specific embodiment of the present invention, as shown in Figs. 2 and 3, a lentivirus vector into which a polynucleotide encoding CD47-CAR was inserted was prepared, and as shown in Fig. 4, anti-CD47 CAR was normally expressed and was confirmed to bind to the CD47 peptide.

Biological methods for introducing polynucleotides into host cells include the use of DNA and RNA vectors. Viral vectors, and in particular retroviral vectors, have become the most widely used methods for inserting genes into mammalian, e.g., human cells. Other virus vector may be derived from lentiviruses, poxviruses, herpes simplex viruses, adenoviruses and adeno-associated viruses, and the like.

Chemical means for introducing polynucleotides into host cells include colloidal dispersion systems such as macromolecular complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g., an artificial membrane vesicle).

When a non-viral delivery system is used, an exemplary delivery vehicle is a liposome. The use of lipid preparations is contemplated for the introduction of nucleic acids into host cells (in vitro, ex vivo or in vivo). In another aspect, the nucleic acid may be associated with a lipid. Nucleic acids associated with lipids may be encapsulated within the aqueous interior of the liposome, interspersed within the lipid bilayer of the liposome, attached to the liposome via a linking molecule associated with both the liposome and oligonucleotide, captured within the liposome, complexed with the liposome, dispersed in a lipid-containing solution, mixed with a lipid or combined with a lipid, contained as a suspension in a lipid, contained or complexed with micelles, or otherwise associated with a lipid. Lipid, lipid/DNA or lipid/expression a vector association composition is not limited to any particular structure in solution.

### Immune effector cell expressing Chimeric antigen receptor (CAR)

In another aspect, the present invention relates to an immune effector cell expressing the chimeric antigen receptor (CAR), and includes a vector comprising a polynucleotide encoding a chimeric antigen receptor (CAR), or a polynucleotide encoding a chimeric antigen receptor (CAR).

In the present invention, the immune effector cell may be a mammalian-derived cell, preferably a T cell or a natural killer (NK) cell.

In the present invention, an immune effector cell expressing the chimeric antigen receptor (CAR) can be prepared by introducing the CAR vector of the present invention into an immune effector cell, for example, a T cell or NK cell.

Specifically, CAR vector can be introduced into cells by methods known in the art, such as electroporation, lipofectamine (lipofectamine 2000, Invitrogen), and the like. For example, an immune effector cell can be transformed by a lentiviral vector to integrate the viral genome carrying the CAR molecule into the host genome to ensure long-term and stable expression of the target gene. For another example, a transposon can be used to introduce a CAR transport plasmid and a transferase transport plasmid into a target cell. For another example, a CAR molecule can be added to the genome by a gene editing method (e.g., CRISPRCas9).

An immune effector cell for the production of immune effector cell expressing a chimeric antigen receptor (CAR) can be obtained from a subject, wherein the "subject" includes a living organism (e.g., a mammal from which an immune response can be elicited). Examples of subjects include humans, dogs, cats, mice, rats, and transgenic species thereof. T cells can be obtained from numerous sources, including peripheral blood mononuclear cells, bone marrow, lymph node tissue, umbilical cord blood, thymus tissue, tissue from the site of infection, ascites, pleural effusion, splenic tissue, and tumors.

Such T cells can be obtained from blood units collected from a subject using any of a number of techniques known to those of ordinary skill in the art, for example, Ficoll^{™} isolation. Cells from blood are obtained by apheresis, and apheresis products typically contain T cells, monocytes, granulocytes, lymphocytes including B cells, other nucleated leukocytes, red blood cells, and platelets.

Cells collected by apheresis can be washed to remove the plasma fraction and place the cells in an appropriate buffer or medium for subsequent processing steps. T cells are isolated from peripheral blood lymphocytes by lysing red blood cells and depleting monocytes, for example by centrifugation through a PERCOLL^{™} gradient or by countercurrent centrifugation.

### Composition for preventing or treating diseases mediated by CD47 expression

In another aspect, the present invention includes a pharmaceutical composition for use in preventing or treating a cancer or tumor expressing CD47, comprising: the antibody specifically binding to CD47 or the fragment thereof; or the immune effector cell expressing a chimeric antigen receptor targeting CD47.

In the present invention, the cancer or tumor expressing CD47 is hematological cancer, ovarian cancer, colon cancer, breast cancer, lung cancer, myeloma, neuroblast-derived CNS tumor, monocytic leukemia, B-cell leukemia, T-cell leukemia leukemia, B-cell lymphoma, T-cell lymphoma, and mast cell tumor.

In the present invention, the composition may include a therapeutic agent for a disease mediated by cells expressing CD47, wherein the therapeutic agent may be covalently bound to the heavy and/or light chain of antibody that specifically binds to CD47, it can be administered in combination with antibody or CD47-CAR-T cell specific for CD47 of the present invention

The therapeutic agent includes a small molecule drug, a peptide drug, a toxin (e.g., a cytotoxin), and the like.

In addition, the therapeutic agent may be an anticancer agent. Anticancer agents reduce the proliferation of cancer cells and include non-peptidyl (i.e., non-protein) compounds, including cytotoxic agents and cytostatic agents. Non-limiting examples of anticancer agents include alkylating agents, nitrosourea, antimetabolites, antitumor antibiotics, plant (vinca) alkaloids, and steroid hormones. Peptide compounds may also be used.

In the pharmaceutical composition, the antibody that specifically binds to CD47 or an immune effector cell expressing a chimeric antigen receptor targeting CD47 may be the only active ingredient in the composition for treatment or diagnosis, or, can be used with other active ingredients for example, an anti-T cell, other antibody components such as anti-IFNy or anti-LPS antibody, or non-antibody components such as xanthine.

The pharmaceutical composition preferably contains a therapeutically effective amount of antibody of the present invention. As used herein, the term "therapeutically effective amount" refers to an amount of a therapeutic agent required to treat, ameliorate, or prevent a target disease or condition, or the amount of a therapeutic agent required to exhibit a detectable therapeutic or prophylactic effect. For any antibody, a therapeutically effective dose can be initially determined by cell culture assays or animal models, usually rodents, rabbits, dogs, pigs, or primates. Animal models can also be used to determine appropriate concentration ranges and routes of administration. Such information can be used to determine useful dosages and routes for dosing in humans.

The precise effective amount for a human patient can vary depending on the severity of the disease state, the patient's general health, the patient's age, weight and sex, diet, administration time, administration frequency, drug composition, response sensitivity, and tolerance/response to treatment. The amount can be determined by routine experimentation and is within the scope of the clinician's judgment. In general, an effective dosage is 0.01-50 mg/kg, preferably 0.1-20 mg/kg, more preferably about 15 mg/kg.

The compositions may be administered to the patient individually or in combination with other preparations, agents, or hormones.

The dosage at which the antibody of the present invention is administered depends on the nature of the condition to be treated, the grade of malignant lymphoma or leukemia, and whether the antibody is used to prevent disease or to treat an existing condition.

The frequency of administration depends on the half-life of the antibody molecule and the duration of the drug's effect. If an antibody molecule has a short half-life (e.g., 2 to 10 hours), it may be necessary to provide one or more doses per day. Alternatively, if an antibody molecule has a long half-life (e.g., 2 to 15 days), it may be necessary to provide a dose once a day, once a week, or once every 1 or 2 months.

In addition, the pharmaceutical composition may contain a pharmaceutically acceptable carrier for administration of an antibody. The carrier itself must not cause the production of an antibody that is harmful to the subject receiving the composition, and must be non-toxic. Suitable carriers may be slowly metabolized macromolecules, such as proteins, polypeptides, liposomes, polysaccharides, polylactic acid, polyglycolic acid, amino acid polymers, amino acid copolymers and inactive viral particles.

A pharmaceutically acceptable salt may be used, and it contains for example, mineral acid salts such as hydrochloride, hydrobromide, phosphate and sulfate, or salts of organic acids such as acetic acid, propionic acid, malonic acid and benzoic acid.

A pharmaceutically acceptable carrier in therapeutic compositions may additionally include liquids such as water, saline, glycerol and ethanol. Additionally, auxiliary substances such as wetting agents, emulsifying agents or pH buffering agents may be present in such compositions. The carrier may be formulated as tablets, pills, sugar-coated tablets, capsules, liquids, gels, syrups, slurries and suspensions for ingestion of the pharmaceutical composition by a patient.

Preferred forms for administration may include those suitable for parenteral administration, for example by injection or infusion. When the product is intended for infusion or injection, it may take the form of suspensions, solutions or emulsions in oil or water-soluble excipients, which may contain prescription agents such as suspending, preservative, stabilizing and/or dispersing agents. Alternatively, an antibody molecule may be in anhydrous form and reconstituted with an appropriate sterile solution prior to use.

Once formulated, the compositions of the present invention can be administered directly to a patient. The patients to be treated may be animals. However, the composition is preferably adapted for administration to human patients.

The pharmaceutical composition of the present invention is not limited, but oral, intravenous, intramuscular, intraarterial, intramedullary, intrathecal, intraventricular, transdermal, transcutaneous, subcutaneous, intraperitoneal, intranasal, intestinal, topical, sublingual, intravaginal or rectal routes. Typically, the therapeutic composition may be prepared as injectable forms as liquid solutions or suspensions. In addition, solid forms suitable for solution or suspension in liquid excipients prior to injection may be prepared.

Direct delivery of the composition may generally be achieved by injection, subcutaneous injection, intraperitoneal injection, intravenous injection, intramuscular injection, or may be delivered to the interstitial space of a tissue. In addition, the composition may be administered to the wound site. Dosage treatment may be a single dose schedule or a multiple dose schedule.

### Diagnosis or monitoring of diseases mediated by cells expressing CD47

In another aspect, the present invention relates to a composition for diagnosing or monitoring a disease mediated by cells expressing CD47, comprising the antibody that specifically binds to CD47.

The antibody that specifically binds to CD47 may be directly or indirectly labeled. An indirect label includes a secondary antibody comprising a detectable label, wherein the secondary antibody binds to an antibody that specifically binds to CD47. Another indirect label includes biotin, wherein an antibody that specifically binds to biotinylated CD47 can be detected using avidin or streptavidin containing a detectable label.

A suitable detectable label includes any composition detectable by spectroscopic, photochemical, biochemical, immunochemical, electrical, optical or chemical means. A suitable labels includes, but are not limited to, magnetic beads, fluorescent dyes (e.g., fluorescein isothiocyanate, Texas red, rhodamine, green fluorescent protein, red fluorescent protein, yellow fluorescent protein, etc.), radioactive labels (e.g., For example, ³H, ¹²⁵I, ³⁵S, ¹⁴C or ³²P), enzymes (e.g., mustard radish peroxidase, alkaline phosphatase, luciferase and the ones commonly used for enzyme-linked immunosorbent assay (ELISA)) and colorimetric labels such as colloidal gold or tinted glass or plastic (e.g., polystyrene, polypropylene, latex, etc.) beads.

In addition, for diagnosis or monitoring, the antibody may be labeled with a fluorescent protein, and may contain a contrast agent or a radioisotope.

When the antibody that specifically binds to CD47 of the present invention is used in a diagnostic kit, the antibody is immobilized on a support, and the support may be a microplate, microarray, chip, glass, bead or particle, or a membrane.

Hereinafter, preferred examples are presented to help the understanding of the present invention. However, the following examples are only provided for easier understanding of the present invention, and the contents of the present invention are not limited by the following examples.

### Example 1: Preparation and selection of the antibody that specifically binds to CD47

To select the antibody specific for CD47 peptide, a hybridoma producing the antibody that can bind to CD47 was prepared and the antibody was selected.

First, splenocytes were extracted by immunization with CD47 protein (Acrobiosystems, cat#CD7-HA2E9), and hybridoma cells were prepared through cell fusion with mouse myeloma cells and splenocytes.

Mouse myeloma cells for cell fusion cannot survive in HAT medium because they do not have HGPRT (HypoxanthineGuanidine-Phosphoribosyl-Transferase), but hybridomas can survive in HAT medium by fusion with splenocytes. Since only hybridomas could be grown using this, it is usually grown in HAT medium until hybridomas were established.

The limiting dilution method was used to select hybridomas that produced the antibody that binds to CD47 from among the grown hybridomas. First, it was made to be less than one cell per 96 well, and then, it was confirmed by ELISA whether the antibody obtained from clones proliferated from one cell binds to CD47, and clones that bind to CD47 were selected. The above process was repeated three times to select hybridomas producing the antibody that binds to CD47. In this way, seven types of antibodies that bind to CD47 were obtained.

The seven types of antibodies were named 7C7, 5H4, 5A4, 4E12, 3H3, 3A5 and 1E7, respectively, and their base and amino acid sequences were analyzed. Sequence information for a heavy chain variable region and a heavy chain variable region of each antibody according to the sequencing result was shown in Tables 1 to 7 below, and the underlined parts in Tables 1 to 7 are complementary determining regions (CDR).

**[Table 1]**

| | Sequence information of 7C7 antibody | | |
|---|---|---|---|
| 7C7 | | sequence information | SEQ ID NO: |
| Heavy chain variable region CDR1 | | GYTFSYV | SEQ ID NO: 1 |
| Heavy chain variable region CDR2 | | INPYNDGT | SEQ ID NO: 2 |
| Heavy chain variable region CDR3 | | ARGRNRYDSWFAY | SEQ ID NO: 3 |
| Light chain variable region CDR1 | | QDISNY | SEQ ID NO: 4 |
| Light chain variable region CDR2 | | YTS | SEQ ID NO: 5 |
| Light chain variable region CDR3 | | QQGNTLPWT | SEQ ID NO: 6 |
| | amino acid sequence of heavy chain variable region | EVQLQQSGPELVKPGASVRMSCKASGYTFTSYVMHWV KQKPGQGLEWIGYINPYNDGTKYNEKFKGKSTLTSDKSS STAYMELSSLTSEDSAVYCCARGRNRYDSWFAYWGQG TLVTVSA | SEQ ID NO: 7 |
| | amino acid sequence of light chain variable region | DIQMTQTTSSLSASLGDRVTISCRASQDISNYLNWYQQK PDGTVKLLIYYTSRLHSGVPSRFSGSGSGTDYSLTISNLEQ EDIATYFCQQGNTLPWTFGGGTKLEIK | SEQ ID NO: 8 |
| | nucleotide sequence of heavy chain variable region | gaggtccagctgcaacagtctggacctgagctggtaaagcctggggcc tcagtgaggatgtcctgcaaggcttctggatacacattcactagctatg ttatgcactgggtgaagcagaagcctgggcagggccttgagtggattg gatatattaatccttacaatgatggtactaagtacaatgagaagttcaa aggcaagtccacactgacttcagacaaatcctccagcacagcctacat ggagctcagcagcctgacctctgaggactctgcggtctattgctgtgca agagggaggaataggtacgactcctggtttgcttactggggccaaggg actctggtcactgtctctgca | SEQ ID NO: 9 |
| | nucleotide sequence of light chain variable region | gatatccagatgacacagactacatcctccctgtctgcctctctgggag acagagtcaccatcagttgcagggcaagtcaggacattagcaattatt taaactggtatcagcagaaaccagatggaactgttaaactcctgatct actacacatcaagattacactcaggagtcccatcaaggttcagtggca gtgggtctggaacagattactctctcaccattagcaacctggagcaag aagatattgccacttacttttgccaacagggtaatacgcttccgtggac gttcggtggaggcaccaagctggaaatcaaa | SEQ ID NO: 10 |

**[Table 2]**

| | Sequence information of 5H4 antibody | | |
|---|---|---|---|
| 5H4 | | sequence information | SEQ ID NO: |
| Heavy chain variable region CDR1 | | GYTFTNYW | SEQ ID NO: 11 |
| Heavy chain variable region CDR2 | | IDPSDSYT | SEQ ID NO: 12 |
| Heavy chain variable region CDR3 | | TRGGKRAMDY | SEQ ID NO: 13 |
| Light chain variable region CDR1 | | QSLVHSNGNTY | SEQ ID NO: 14 |
| Light chain variable region CDR2 | | KVS | SEQ ID NO: 15 |
| | Light chain variable region CDR3 | SQSTHVPFT | SEQ ID NO: 16 |
| | amino acid sequence of heavy chain variable region | QVQLQQPGAELVKPGTSVKMSCKAS GYTFTNYWMHWVKQRPGQVLEWIGV IDPSDSYTSYNQKFKGKATLTVDTSSTTAYMQLSSLTSED SAVYYC TRGGKRAMDYWGQGTSVTVSS | SEQ ID NO: 17 |
| | amino acid sequence of light chain variable region | DVLMTQTPLSLPVSLGDQASISCRSSQSLVHSNGNTYLH WYLQKPGQSPKLLIYKVSNRFSGVPDRFSGSGSGTDFTL KISRVEAEDLGVYFCSQSTHVPFTFGSGTKLEIK | SEQ ID NO: 18 |
| | nucleotide sequence of heavy chain variable region | caggtccaactgcagcagcctggggctgagctggtgaagcctgggact tcagtgaagatgtcctgcaaggcttctggctacaccttcaccaactact ggatgcactgggtgaagcagaggcctggacaagtccttgagtggatc ggagtgattgatccttctgatagttatactagctacaatcaaaagttca agggcaaggccacattgactgtagacacatcctccaccacagcctaca tgcagctcagcagcctgacatctgaggactctgcggtctattactgtac aagagggggtaagagagctatggactactggggtcaaggaacctcag tcaccgtctcctca | SEQ ID NO: 19 |
| | nucleotide sequence of light chain variable region | gatgttttgatgacccaaactccactctccctgcctgtcagtcttggaga tcaagcctccatctcttgcagatctagtcagagccttgtacacagtaat ggaaacacctatttacattggtacctgcagaagccaggccagtctcca aagctcctgatctacaaagtttccaaccgattttctggggtcccagaca ggttcagtggcagtggatcagggacagatttcacactcaagatcagca gagtggaggctgaggatctgggagtttatttctgctctcaaagtacaca tgttccattcacgttcggctcggggacaaagttggaaataaaa | SEQ ID NO: 20 |

**[Table 3]**

| | Sequence information of 5A4 antibody | | |
|---|---|---|---|
| 5A4 | | sequence information | SEQ ID NO: |
| Heavy chain variable region CDR1 | | GYTFTNYW | SEQ ID NO: 21 |
| Heavy chain variable region CDR2 | | IDPSNSAT | SEQ ID NO: 22 |
| Heavy chain variable region CDR3 | | ARGGFAFDS | SEQ ID NO: 23 |
| | Light chain variable region CDR1 | QSLVHSNGNTY | SEQ ID NO: 24 |
| | Light chain variable region CDR2 | KVS | SEQ ID NO: 25 |
| | Light chain variable region CDR3 | SQSTHVPWT | SEQ ID NO: 26 |
| | amino acid sequence of heavy chain variable region | QVQLQQPGPELVRPGASVKMSCKAS GYTFTNYWIHWVKQRPGQGLEWIGM IDPSNSATRLNQKFKDKATLNVDKSSNTAYMQLSSLTS EDSAVYYCARGGFAFDSWGQGTTLTVSS | SEQ ID NO: 27 |
| | amino acid sequence of light chain variable region | DVLMTQTPLSLPVSLGDQASISCRSSQSLVHSNGNTYL HWYLQKPGQSPKLLIYKVSNRFSGVPDRFSGSGSGTDF TLKISRVEAEDLGVYFCSQSTHVPWTFGGGTKLEIK | SEQ ID NO: 28 |
| | nucleotide sequence of heavy chain variable region | caggtccaactgcagcagcctgggcctgagctggtgaggcctgggg cttcagtgaagatgtcctgcaaggcttcaggctataccttcaccaact actggattcactgggtgaaacagaggcctggacaaggccttgagtg gattggcatgattgatccttccaatagtgcaactaggttaaatcaga agttcaaggacaaggccacattgaatgtagacaaatcctccaacac agcctacatgcagctcagcagcctgacatctgaggactctgcagtct attactgtgcaagaggagggttcgcctttgactcctggggccaaggc accactctcacagtctcctca | SEQ ID NO: 29 |
| | nucleotide sequence of light chain variable region | gatgttttgatgacccaaactccactctccctgcctgtcagtcttggag atcaagcctccatctcttgcagatctagtcagagccttgtacatagta atggaaacacctatttacattggtacctgcagaagccaggccagtct ccaaagctcctgatctacaaagtttccaaccgattttctggggtccca gacaggttcagtggcagtggatcagggacagatttcacactcaaga tcagcagagtggaggctgaggatctgggagtttatttctgctctcaaa gtacacatgttccgtggacgttcggtggaggcaccaagctggaaatc aaa | SEQ ID NO: 30 |

**[Table 4]**

| | Sequence information of 4E12 antibody | | |
|---|---|---|---|
| 4E12 | | sequence information | SEQ ID NO: |
| Heavy chain variable region CDR1 | | GYTFTNYG | SEQ ID NO: 31 |
| Heavy chain variable region CDR2 | | INTYTGEP | SEQ ID NO: 32 |
| Heavy chain variable region CDR3 | | ARGGGRGAMDY | SEQ ID NO: 33 |
| Light chain variable region CDR1 | | QSIVHSNGNTY | SEQ ID NO: 34 |
| | Light chain variable region CDR2 | KVS | SEQ ID NO: 35 |
| | Light chain variable region CDR3 | FQGSHVPFT | SEQ ID NO: 36 |
| | amino acid sequence of heavy chain variable region | QIQFAQSGPELKKSGETVKISCRAS GYTFTNYGMNWVKQAPGKGLKWMGW INTYTGEPTYADDFKGRFAFSLETSASTAYLQINNLKN EDMATYFCARGGGRGAMDYWGQGTTLTVSS | SEQ ID NO: 37 |
| | amino acid sequence of light chain variable region | DVLMTQTPLSLPVSLGDQASISCRSSQSIVHSNGNTY LDWYLQKPGQSPKLLIYKVSKRFSGVPDRFSGSGSGT DFTLKISRVEAEDLGVYYCFQGSHVPFTFGSGTKLEIK | SEQ ID NO: 38 |
| | nucleotide sequence of heavy chain variable region | cagatccagttcgcgcagtctggacctgagctgaagaagtctgga gagacagtcaagatctcctgcagggcttctgggtataccttcacaa actatggaatgaactgggtgaagcaggctccaggaaagggtttaa agtggatgggctggataaacacctacactggagagccaacatatg ctgatgacttcaagggacggtttgccttctctttggaaacctctgcc agcactgcctatttgcagatcaacaacctcaaaaatgaggacatg gctacatatttctgtgcaagagggggtgggaggggtgctatggact actggggccaaggcaccactctcacagtctcctca | SEQ ID NO: 39 |
| | nucleotide sequence of light chain variable region | gatgttttgatgacccaaactccactctccctgcctgtcagtcttgg agatcaagcctccatctcttgcagatctagtcagagcattgtacat agtaatggaaacacctatttagactggtacctgcagaaaccaggc cagtctccaaagctcctgatctacaaagtttccaaacgattttctgg ggtcccagacaggttcagtggcagtggatcagggacagatttcac actcaagatcagcagagtggaggctgaggatctgggagtttatta ctgctttcaaggttcacatgttccattcacgttcggctcggggacaa agttggaaataaaa | SEQ ID NO: 40 |

**[Table 5]**

| | Sequence information of 3H3 antibody | | |
|---|---|---|---|
| 3H3 | | sequence information | SEQ ID NO: |
| Heavy chain variable region CDR1 | | GYTFTNYW | SEQ ID NO: 41 |
| Heavy chain variable region CDR2 | | IDPSNSET | SEQ ID NO: 42 |
| Heavy chain variable region CDR3 | | ARGGFAFDS | SEQ ID NO: 43 |
| Light chain variable region CDR1 | | QSLVHNNGNTY | SEQ ID NO: 44 |
| Light chain variable region CDR2 | | KVS | SEQ ID NO: 45 |
| | Light chain variable region CDR3 | SQSTHVPWT | SEQ ID NO: 46 |
| | amino acid sequence of heavy chain variable region | EVQLQQPGPELVRPGASVKMSCKAS GYTFTNYWMHWVKQRPGQGLEWIGM IDPSNSETRLNQKFKDKATLNVDKSSNTAYMQLSSLT SEDSAVYSCARGGFAFDSWGQGTTLTVSS | SEQ ID NO: 47 |
| | amino acid sequence of light chain variable region | DVLMTQTPLSLPVSLGDQASISCRSSQSLVHNNGNT YLHWYLQKPGQSPKLLIYKVSNRFSGVPDRFRGSGS GTDFTLKISRVEAEDLGVYFCSQSTHVPWTFGGGTKL EIK | SEQ ID NO: 48 |
| | nucleotide sequence of heavy chain variable region | gaggtccagctgcagcagcctgggcctgagctggtgaggcctggg gcttcagtgaagatgtcctgcaaggcttcaggctataccttcacca actactggatgcactgggtgaaacagaggcctggacaaggccttg agtggattggcatgattgatccttccaatagtgaaactaggttaaa tcagaagttcaaggacaaggccacattgaatgtagacaaatcctc caacacagcctacatgcagctcagcagcctgacatctgaggactc tgcagtctattcctgtgcaagaggagggttcgcctttgactcctggg gccaaggcaccactctcacagtctcctca | SEQ ID NO: 49 |
| | nucleotide sequence of light chain variable region | gatgttttgatgacccaaactccactctccctgcctgtcagtcttgg agatcaagcctccatctcttgcagatctagtcagagccttgtacac aataatggaaacacctatttacattggtacctgcagaagccaggc cagtctccaaagctcctgatctacaaagtttccaaccgattttctgg ggtcccagacaggttccgtggcagtggatcggggacagatttcac actcaagatcagcagagtggaggctgaggatctgggagtttatttc tgctctcaaagtacacatgttccgtggacgttcggtggaggcacca agctggaaatcaaa | SEQ ID NO: 50 |

**[Table 6]**

| | Sequence information of 3A5 antibody | | |
|---|---|---|---|
| 3A5 | | sequence information | SEQ ID NO: |
| Heavy chain variable region CDR1 | | GYTFSYW | SEQ ID NO: 51 |
| Heavy chain variable region CDR2 | | IDPSDSYT | SEQ ID NO: 52 |
| Heavy chain variable region CDR3 | | ARGGKRAMDY | SEQ ID NO: 53 |
| Light chain variable region CDR1 | | QSLVHSNGNTY | SEQ ID NO: 54 |
| | Light chain variable region CDR2 | KVS | SEQ ID NO: 55 |
| | Light chain variable region CDR3 | SQSTHVPFT | SEQ ID NO: 56 |
| | amino acid sequence of heavy chain variable region | EVQLQQPGAELVKPGASVKMSCKAS GYTFTSYWMHWMNQRPGQGLEWIGV IDPSDSYTSYNQKFKGKATLTVDTSSSTAYMQLSSLTSE DSAVYYCARGGKRAMDYWGQGTSVTVSS | SEQ ID NO: 57 |
| | amino acid sequence of light chain variable region | DVLMTQTPLSLPVSLGDQASISCRSSQSLVHSNGNTYL HWYLQKPGQSPKLLIYKVSNRFSGVPDRFSGSGSGTDF TLKISRVEAEDLGVYFCSQSTHVPFTFGSGTKLEIK | SEQ ID NO: 58 |
| | nucleotide sequence of heavy chain variable region | gaggtccagctgcagcagcctggggctgagctggtgaagcctgggg cttcagtgaagatgtcctgcaaggcttctggctacaccttcaccagct actggatgcactggatgaaccagaggcctggacaaggccttgagtg gatcggagtgattgatccttctgatagttatactagctacaatcaaaa gttcaagggcaaggccacattgactgtagacacatcctccagcaca gcctacatgcagctcagcagcctgacatctgaggactctgcggtcta ttactgtgcaagagggggtaagagagctatggactactggggtcaa ggaacctcagtcaccgtctcctca | SEQ ID NO: 59 |
| | nucleotide sequence of light chain variable region | gatgttttgatgacccaaactccactctccctgcctgtcagtcttggag atcaagcctccatctcttgcagatctagtcagagccttgtacacagta atggaaacacctatttacattggtacctgcagaagccaggccagtct ccaaagctcctgatctacaaagtttccaaccgattttctggggtccca gacaggttcagtggcagtggatcagggacagatttcacactcaaga tcagcagagtggaggctgaggatctgggagtttatttctgctctcaaa gtacacatgttccattcacgttcggctcggggacaaagttggaaata aaa | SEQ ID NO: 60 |

**[Table 7]**

| | Sequence information of 1E7 antibody | | |
|---|---|---|---|
| 1E7 | | sequence information | SEQ ID NO: |
| Heavy chain variable region CDR1 | | GYIFTSYV | SEQ ID NO: 61 |
| Heavy chain variable region CDR2 | | INPYNDGT | SEQ ID NO: 62 |
| Heavy chain variable region CDR3 | | ARGGFTTDY | SEQ ID NO: 63 |
| Light chain variable region CDR1 | | QSLVHSNGNTY | SEQ ID NO: 64 |
| | Light chain variable region CDR2 | KVS | SEQ ID NO: 65 |
| | CDR3 on the light chain variable region | SQSTHVPYT | SEQ ID NO: 66 |
| | amino acid sequence of heavy chain variable region | EVQLQQSGPELIKPGASVKMSCKAS GYIFTSYVVYWVKQKPGQGLEWIGY INPYNDGTKYNEKFKGKATLTSYKSSSTAYMELSSLTSA DSAVYYCARGGFTTDYWGQGTTLTVSS | SEQ ID NO: 67 |
| | amino acid sequence of light chain variable region | DVLMTQTPLSLPVSLGDQASISCRSSQSLVHSNGNTYL HWYLQKPGQSPKLLIYKVSNRFSGVPDRFSGSGSGTDF TLKISRVEAEDLGVYFCSQSTHVPYTFGGGTKLEIK | SEQ ID NO: 68 |
| | nucleotide sequence of heavy chain variable region | gaggtccagctgcaacagtctggacctgagctgataaagcctgggg cttcagtgaagatgtcctgcaaggcttctggatacatattcactagtt atgttgtgtattgggtgaagcagaagcctgggcagggccttgagtgg attggatatattaatccttacaatgatggtactaagtacaatgagaa gttcaagggcaaggccacactgacttcatacaaatcctccagcaca gcctacatggagctcagcagcctgacctctgcggactctgcggtctat tactgtgcaagaggggggtttactactgactactggggccaaggcac cactctcacagtctcctca | SEQ ID NO: 69 |
| | nucleotide sequence of light chain variable region | gatgttttgatgacccaaactccactctccctgcctgtcagtcttggag atcaagcctccatctcttgcagatctagtcagagccttgtacacagta atggaaacacctatttacattggtacctgcagaagccaggccagtct ccaaagctcctgatctacaaagtttccaaccgattttctggggtccca gacaggttcagtggcagtggatcagggacagatttcacactcaaga tcagcagagtggaggctgaggatctgggagtttatttctgctctcaaa gtacacatgttccttacacgttcggaggggggaccaagctggaaata aaa | SEQ ID NO: 70 |

### Example 2: Confirmation of specificity of the selected antibody for CD47 - ELISA analysis

In the present invention, in order to confirm the specificity of 7C7, 5H4, 5A4, 4E12, 3H3, 3A5 and 1E7 antibodies established in Example 1 for CD47, ELISA analysis was performed.

First, in order to encode the CD47 peptide, CD47 protein (Acrobiosystems, cat#CD7-HA2E9) was dispensed in a 96-well plate at a concentration of 100 ng/well, and then reacted at 4°C overnight. Then, after treatment with 1 X PBST containing 3% BSA, blocking at room temperature for 30 minutes.

3 µl of hybridoma cell culture solution of each clone for producing 7C7, 5H4, 5A4, 4E12, 3H3, 3A5, or 1E7 antibody was treated in each well, and then reacted at room temperature for 2 hours, and then washed 3 times with 1 X PBST. Secondary antibody (anti-HRP, 1:10,000) was treated and reacted at room temperature for 30 minutes, washed 3 times with 1 X PBST, and then treated with TMB for color development and reacted at room temperature for 5 minutes. Finally, the reaction was terminated by treatment with a stop solution of 1N H₂SO₄, and then the absorbance was measured at 450 nm.

**[Table 8]**

| | ELISA Experimental Conditions | |
|---|---|---|
| ELISA reader | | Infinite F50 |
| Measurement Filter | | 450 nm |
| Measurement Mode | | Single Point Photo |
| Antigen Coating | | 100 ng/well |
| 2nd Antibody (Anti-mlgG-HRP) | | 1:10,000 dilution |
| Substrate | | TMB |

**[Table 9]**

| | ELISA test results | |
|---|---|---|
| antibody type | | OD450 measurements |
| 7C7 | | 1.980 |
| 5H4 | | 1.914 |
| 5A4 | | 1.932 |
| 4E12 | | 2.155 |
| 3H3 | | 1.966 |
| 3A5 | | 2.010 |
| 1E7 | | 2.137 |

As a result, as shown in Table 9, it was confirmed that all of the antibodies selected in the present invention specifically bound to CD47.

### Example 3: Confirmation of specificity of the selected antibody for CD47 - flow cytometer

To confirm the specificity of 5H4, 5A4, 4E12, 3H3, 3A5 and 1E7 antibodies for CD47 established in Example 1, flow cytometer was performed.

First, 1 × 10⁷ of breast cancer cell line MCF-7 expressing CD47 and 1 µg of 7C7, 5H4, 5A4, 4E12, 3H3, 3A5, and 1E7 antibody were reacted for 30 minutes, respectively, and then stained on surface with a secondary antibody. After staining, it was measured by flow cytometry.

As a positive control, CD47 antibody (Biolegend PE anti-human CD47, cat# 323108, 5 µl) was used, and as a secondary antibody, PE-conjugated anti-mouse IgG antibody (PE-conjugated goat anti- mouse IgG; Biolegend Inc., cat# 405307, USA, 5 µl) was used.

**[Table 10]**

| | Flow cytometry results | | | |
|---|---|---|---|---|
| antibody type | | Count | Median | Mean |
| 7C7 | | 11544 | 6113 | 6816 |
| 5H4 | | 11244 | 14313 | 15493 |
| 5A4 | | 11298 | 14668 | 15992 |
| 4E12 | | 11239 | 5385 | 6026 |
| 3H3 | | 11164 | 16536 | 18001 |
| 3A5 | | 11285 | 13866 | 14951 |
| 1E7 | | 11231 | 14921 | 16140 |
| positive | | 11535 | 10972 | 11930 |
| 2nd Ab alone | | 11285 | 73.9 | 80 |
| none | | 11077 | 24.5 | 25.0 |

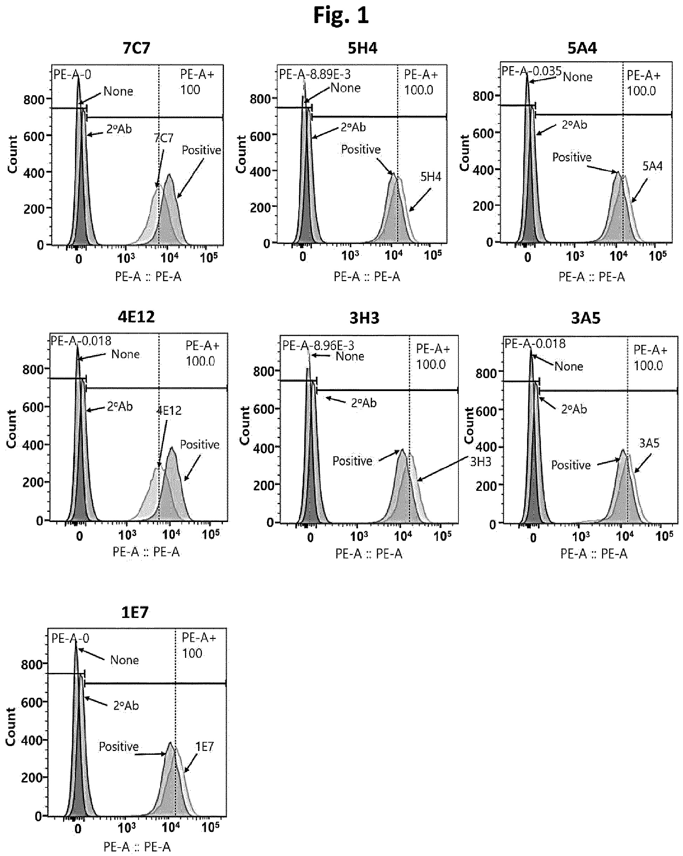

As a result, as shown in FIG. 1 and Table 10, it was confirmed that all of the 7C7, 5H4, 5A4, 4E12, 3H3, 3A5 and 1E7 antibodies specifically bound to CD47-expressing cells.

Since the antibody selected in the present invention specifically recognized CD47-expressing cells, it can effectively induce cytotoxicity or death by immune cells/macrophages by inhibiting immune evasion of CD47-expressing cancer or tumor cells. Therefore, the anti-CD47 antibody of the present invention can be usefully used as a composition for preventing or treating a cancer or tumor expressing CD47.

### Example 4: Vector construction expressing chimeric antigen receptor targeting CD47 (CD47-CAR)

In the present invention, a lentivirus vector (CD47-CAR lentivirus) expressing a chimeric antigen receptor (CAR) targeting CD47 was prepared using the CD47 antibody prepared in Example 1.

As shown in the schematic diagram of Figure 2, CAR DNA composed of:
EFla promoter (SEQ ID NO: 87);
a polynucleotide encoding a signal peptide (SEQ ID NO: 88);
a polynucleotide encoding the CD47-binding domain (7C7 antibody represented by the nucleotide sequence of SEQ ID NO: 74, 5H4 antibody represented by the nucleotide sequence of SEQ ID NO: 76, 5A4 antibody represented by the nucleotide sequence of SEQ ID NO: 78, 4E12 antibody represented by the nucleotide sequence of SEQ ID NO: 80, 3H3 antibody represented by the nucleotide sequence of SEQ ID NO: 82, 3A5 antibody represented by the nucleotide sequence of SEQ ID NO: 84 or 1E7 antibody represented by the nucleotide sequence of SEQ ID NO: 86);
a polynucleotide encoding the CD8 hinge region (SEQ ID NO: 89);
a polynucleotide encoding a transmembrane domain (SEQ ID NO: 90);
a polynucleotide encoding 4-1BB (costimulatory domain) (SEQ ID NO: 91);
a polynucleotide encoding CD3ζ intracellular signal transduction domain (SEQ ID NO: 92); and
a polynucleotide (SEQ ID NO: 93) encoding WPRE;
was synthesized in vitro and inserted into a 3rd generation lentivirus vector.

Lentivirus vector DNA (0.5 µg) was transferred to HEK293FT cells (5×10⁵ cells/500 µl), and 293HEK cells expressing the CD47-CAR gene were prepared. Lipofectamine 3000 transfection kit (Invitrogen, cat# L3000-015) was used to transfer genes into 293HEK cells, and cultured in Opti-MEM (gibco, cat# 51985-034) medium for 4 hours.

In HEK293FT transformed with lentivirus vector DNA, it was confirmed by flow cytometry whether CD47-specific CAR was normally expressed and bound to CD47 peptide (FIG. 3), as shown in FIG. 4, CD47-CAR was normally expressed and confirmed to bind to the CD47 peptide. Therefore, the anti-CD47 antibody, 5A4, 4E12, 3H3, 3A5 or 1E7, can be used to prepare CAR-T cells targeting CD47.

### [Industrial Applicability]

It was confirmed that seven types of CD47-specific antibodies (7C7, 5H4, 5A4, 4E12, 3H3, 3A5, 1E7) selected in the present invention specifically bound to CD47 antigen, and it is possible to produce a chimeric antigen receptor (CAR) that target CD47 using the established antibody. Thus, the present of CD47-specific antibodies and chimeric antigen receptors prepared by the above antibody can be applied for use in preventing or treating a cancer or tumor expressing CD47.

## Claims

1. An antibody specifically binding to CD47 or a fragment thereof, comprising:
(1) a heavy chain variable region including a CDR1 region represented by an amino acid of SEQ ID NO: 1, a CDR2 region represented by an amino acid of SEQ ID NO: 2 and a CDR3 region represented by an amino acid of SEQ ID NO: 3, and a light chain variable region including a CDR1 region represented by an amino acid of SEQ ID NO: 4, a CDR2 region represented by an amino acid of SEQ ID NO: 5 and a CDR3 region represented by an amino acid of SEQ ID NO: 6;
(2) a heavy chain variable region including a CDR1 region represented by an amino acid of SEQ ID NO: 11, a CDR2 region represented by an amino acid of SEQ ID NO: 12 and a CDR3 region represented by an amino acid of SEQ ID NO: 13, and a light chain variable region including a CDR1 region represented by an amino acid of SEQ ID NO: 14, a CDR2 region represented by an amino acid of SEQ ID NO: 15 and a CDR3 region represented by an amino acid of SEQ ID NO: 16;
(3) a heavy chain variable region including a CDR1 region represented by an amino acid of SEQ ID NO: 21, a CDR2 region represented by an amino acid of SEQ ID NO: 22 and a CDR3 region represented by an amino acid of SEQ ID NO: 23, and a light chain variable region including a CDR1 region represented by an amino acid of SEQ ID NO: 24, a CDR2 region represented by an amino acid of SEQ ID NO: 25 and a CDR3 region represented by an amino acid of SEQ ID NO: 26;
(4) a heavy chain variable region including a CDR1 region represented by an amino acid of SEQ ID NO: 31, a CDR2 region represented by an amino acid of SEQ ID NO: 32 and a CDR3 region represented by an amino acid of SEQ ID NO: 33, and a light chain variable region including a CDR1 region represented by an amino acid of SEQ ID NO: 34, a CDR2 region represented by an amino acid of SEQ ID NO: 35 and a CDR3 region represented by an amino acid of SEQ ID NO: 36;
(5) a heavy chain variable region including a CDR1 region represented by an amino acid of SEQ ID NO: 41, a CDR2 region represented by an amino acid of SEQ ID NO: 42 and a CDR3 region represented by an amino acid of SEQ ID NO: 43, and a light chain variable region including a CDR1 region represented by an amino acid of SEQ ID NO: 44, a CDR2 region represented by an amino acid of SEQ ID NO: 45 and a CDR3 region represented by an amino acid of SEQ ID NO: 46;
(6) a heavy chain variable region including a CDR1 region represented by an amino acid of SEQ ID NO: 51, a CDR2 region represented by an amino acid of SEQ ID NO: 52 and a CDR3 region represented by an amino acid of SEQ ID NO: 53, and a light chain variable region including a CDR1 region represented by an amino acid of SEQ ID NO: 54, a CDR2 region represented by an amino acid of SEQ ID NO: 55 and a CDR3 region represented by an amino acid of SEQ ID NO: 56; or
(7) a heavy chain variable region including a CDR1 region represented by an amino acid of SEQ ID NO: 61, a CDR2 region represented by an amino acid of SEQ ID NO: 62 and a CDR3 region represented by an amino acid of SEQ ID NO: 63, and a light chain variable region including a CDR1 region represented by an amino acid of SEQ ID NO: 64, a CDR2 region represented by an amino acid of SEQ ID NO: 65 and a CDR3 region represented by an amino acid of SEQ ID NO: 66.

2. The antibody specifically binding to CD47 or a fragment thereof of claim 1, wherein the (1) antibody comprises a heavy chain variable region represented by an amino acid of SEQ ID NO: 7 and a light chain variable region represented by an amino acid of SEQ ID NO: 8;
the (2) antibody comprises a heavy chain variable region represented by an amino acid of SEQ ID NO: 17 and a light chain variable region represented by an amino acid of SEQ ID NO: 18;
the (3) antibody comprises a heavy chain variable region represented by an amino acid of SEQ ID NO: 27 and a light chain variable region represented by an amino acid of SEQ ID NO: 28;
the (4) antibody comprises a heavy chain variable region represented by an amino acid of SEQ ID NO: 37 and a light chain variable region represented by an amino acid of SEQ ID NO: 38;
the (5) antibody comprises a heavy chain variable region represented by an amino acid of SEQ ID NO: 47 and a light chain variable region represented by an amino acid of SEQ ID NO: 48;
the (6) antibody comprises a heavy chain variable region represented by an amino acid of SEQ ID NO: 57 and a light chain variable region represented by an amino acid of SEQ ID NO: 58; or
the (7) antibody comprises a heavy chain variable region represented by an amino acid of SEQ ID NO: 67 and a light chain variable region represented by an amino acid of SEQ ID NO: 68.

3. A polynucleotide encoding the antibody specifically binding to CD47 or a fragment thereof of claim 1 or 2.

4. A vector comprising the polynucleotide encoding the antibody specifically binding to CD47 or a fragment thereof of claim 1 or 2.

5. A recombinant cell producing the antibody or fragment thereof specifically binding to CD47 or a fragment thereof transformed with the vector of claim 4.

6. A chimeric antigen receptor (CAR) comprising: a CD47-binding domain; a transmembrane domain; a costimulatory domain; and an intracellular signal transduction domain,
wherein the CD47-binding domain is an antibody specifically binding to CD47 or a fragment thereof, comprising:
(1) a heavy chain variable region including a CDR1 region represented by an amino acid of SEQ ID NO: 1, a CDR2 region represented by an amino acid of SEQ ID NO: 2 and a CDR3 region represented by an amino acid of SEQ ID NO: 3, and a light chain variable region including a CDR1 region represented by an amino acid of SEQ ID NO: 4, a CDR2 region represented by an amino acid of SEQ ID NO: 5 and a CDR3 region represented by an amino acid of SEQ ID NO: 6;
(2) a heavy chain variable region including a CDR1 region represented by an amino acid of SEQ ID NO: 11, a CDR2 region represented by an amino acid of SEQ ID NO: 12 and a CDR3 region represented by an amino acid of SEQ ID NO: 13, and a light chain variable region including a CDR1 region represented by an amino acid of SEQ ID NO: 14, a CDR2 region represented by an amino acid of SEQ ID NO: 15 and a CDR3 region represented by an amino acid of SEQ ID NO: 16;
(3) a heavy chain variable region including a CDR1 region represented by an amino acid of SEQ ID NO: 21, a CDR2 region represented by an amino acid of SEQ ID NO: 22 and a CDR3 region represented by an amino acid of SEQ ID NO: 23, and a light chain variable region including a CDR1 region represented by an amino acid of SEQ ID NO: 24, a CDR2 region represented by an amino acid of SEQ ID NO: 25 and a CDR3 region represented by an amino acid of SEQ ID NO: 26;
(4) a heavy chain variable region including a CDR1 region represented by an amino acid of SEQ ID NO: 31, a CDR2 region represented by an amino acid of SEQ ID NO: 32 and a CDR3 region represented by an amino acid of SEQ ID NO: 33, and a light chain variable region including a CDR1 region represented by an amino acid of SEQ ID NO: 34, a CDR2 region represented by an amino acid of SEQ ID NO: 35 and a CDR3 region represented by an amino acid of SEQ ID NO: 36;
(5) a heavy chain variable region including a CDR1 region represented by an amino acid of SEQ ID NO: 41, a CDR2 region represented by an amino acid of SEQ ID NO: 42 and a CDR3 region represented by an amino acid of SEQ ID NO: 43, and a light chain variable region including a CDR1 region represented by an amino acid of SEQ ID NO: 44, a CDR2 region represented by an amino acid of SEQ ID NO: 45 and a CDR3 region represented by an amino acid of SEQ ID NO: 46;
(6) a heavy chain variable region including a CDR1 region represented by an amino acid of SEQ ID NO: 51, a CDR2 region represented by an amino acid of SEQ ID NO: 52 and a CDR3 region represented by an amino acid of SEQ ID NO: 53, and a light chain variable region including a CDR1 region represented by an amino acid of SEQ ID NO: 54, a CDR2 region represented by an amino acid of SEQ ID NO: 55 and a CDR3 region represented by an amino acid of SEQ ID NO: 56; or
(7) a heavy chain variable region including a CDR1 region represented by an amino acid of SEQ ID NO: 61, a CDR2 region represented by an amino acid of SEQ ID NO: 62 and a CDR3 region represented by an amino acid of SEQ ID NO: 63, and a light chain variable region including a CDR1 region represented by an amino acid of SEQ ID NO: 64, a CDR2 region represented by an amino acid of SEQ ID NO: 65 and a CDR3 region represented by an amino acid of SEQ ID NO: 66.

7. The chimeric antigen receptor of claim 6, wherein the transmembrane domain is a protein selected from the group consisting of CD8α, CD4, CD28, CD137, CD80, CD86, CD152 and PD1,
the costimulatory domain is a protein selected from the group consisting of CD28, 4-1BB, OX-40 and ICOS, and
the intracellular signal transduction domain is CD3ζ.

8. The chimeric antigen receptor of claim 6, further comprising a hinge region between a C-terminus of the CD47-binding domain and an N-terminus of the transmembrane domain.

9. A polynucleotide encoding the chimeric antigen receptor of any one of claims 6 to 8.

10. A vector comprising the polynucleotide encoding the chimeric antigen receptor of any one of claims 6 to 8.

11. An immune effector cell comprising: the vector of claim 10, or the polynucleotide encoding the chimeric antigen receptor of any one of claims 6 to 8.

12. A pharmaceutical composition for use in preventing or treating a cancer or tumor expressing CD47, comprising: the antibody specifically binding to CD47 or the fragment thereof of claim 1 or 2; or
the immune effector cell of claim 11.

13. The pharmaceutical composition for use according to claim 12, wherein the cancer ortumor is selected from the group consisting of blood cancer, ovarian cancer, colon cancer, breast cancer, lung cancer, myeloma, neuroblast-derived CNS cancer, monocytic leukemia, B- cell induced leukemia, T-cell leukemia, B-cell lymphoma, T-cell lymphoma, and mast cell-derived cancer
